# EUROPEAN PATENT APPLICATION

(11) **EP 4 497 400 A1**
(43) Date of publication of application: **29.01.2025**
(21) Application number: 22933343.0
(22) Date of filing: 23.03.2022
(51) Int. Cl.: A61B 17/28

(54) **FORCEPS DEVICE**

(71) Applicant: RIVERFIELD Inc., 1070052 Tokyo (JP)
(72) Inventor: AKIYOSHI, Kazuki, Tokyo 160-0017 (JP)
(74) Representative: adares Patent- und Rechtsanwälte Reininger & Partner GmbB
(86) International application number: PCT/JP2022/013610
(87) International publication number: WO 2023/181201

(57) **Abstract**

A forceps device (10) includes: a distal end part including a treatment part for treating an object; a columnar member connected to the distal end part; a pipe (28) connected to the columnar member on a side thereof opposite the side with which the distal end part is connected; a joint cover (30) mounted over the distal end part to the columnar member; and an enlarged-diameter portion (34) having a diameter (d2) larger than a diameter (d1) of an end face (30a), on a side closer to the pipe, of the joint cover (30) mounted over the columnar member. The enlarged-diameter portion (34) is located at a position covering the end face (30a).

## Description

### Technical Field

The present invention relates to a forceps device.

### Background Art

Medical treatments using robots (manipulators) have recently been proposed in order to reduce the burden on operators and save manpower in medical facilities. In the field of surgery, proposals have been made for surgical manipulator systems for operators to treat patients by operating remotely-controllable surgical manipulators.

Various surgical tools are attached to distal ends of surgical manipulators. Known surgical tools include forceps for grasping human tissue during surgery. For example, Patent Literature 1 teaches a cover for a surgical treatment tool, which is a member having a cylindrical shape to surround at least a base of the surgical treatment tool. The cover for a surgical treatment tool is made of an insulating material.

### Related Art List

### Patent Literature

Patent Literature 1: JP 2019-195402 A

### Summary of Invention

### Technical Problem

The cover for a surgical treatment tool mentioned above, however, is made of a flexible material such as rubber, which can be used during endoscopic surgery or the like. Thus, when the surgical treatment tool is inserted or removed through a trocar, the cover may get caught on an end of the trocar. Thus, in some cases, the cover may come off the surgical treatment tool or may be moved from its correct position. For example, movement of the cover from its correct position on the surgical treatment tool affects the insulation performance.

The present invention has been made in view of the aforementioned circumstances, and an exemplary object thereof is to provide a novel technology that makes a cover less likely to come off a forceps device or move from its correct position.

### Solution to problem

To solve the aforementioned problems, a forceps device according to an aspect of the present invention includes: a distal end part including a treatment part for treating an object; a columnar member connected to the distal end part; a pipe connected to the columnar member on a side thereof opposite the side with which the distal end part is connected; a cover mounted over the distal end part to the columnar member; and an enlarged-diameter portion having a larger diameter than an end face, on a side closer to the pipe, of the cover mounted over the columnar member. The enlarged-diameter portion is located at a position covering the end face.

According to this aspect, the end of the trocar hardly gets caught on the end face of the cover when the forceps device is being removed from or inserted into the trocar. In addition, even if the end of the trocar comes in contact with the cover, the contact position can be shifted from the end face of the cover toward the distal end side.

The enlarged-diameter portion may have a tapered shape with its diameter decreasing from an end adjacent to the columnar member toward an end adjacent to the pipe. This makes the end of the trocar less likely to get caught on the end face of the cover when the forceps device inserted in the trocar is being removed. In addition, the angle between the axis of the trocar and the axis of the forceps device is an acute angle when the forceps device inserted in the trocar is being removed.

A diameter of the enlarged-diameter portion at the end of the tapered shape adjacent to the pipe is smaller than a diameter of the pipe. As a result, the end of the trocar is less likely to get caught on the end, which is adjacent to the pipe, of the enlarged-diameter portion when the forceps device inserted in the trocar is being removed.

The enlarged-diameter portion may be provided at the columnar member or the pipe. This makes the end of the trocar less likely to get caught on the end face of the cover around the region between the columnar member and the pipe.

The diameter of the enlarged-diameter portion on the columnar member side is smaller than an inner diameter of a trocar into which the forceps device is inserted. This enables the forceps device to be inserted into the trocar.

A wire for causing the treatment part to operate may be included. The columnar member may have a through-hole through which the wire extends.

Note that any combination of the components described above, and any expression in the present invention converted to that for a method, a device, a system, and the like also remain as aspects of the present invention.

### Advantageous Effects of Invention

According to the present invention, a cover hardly comes off a forceps device.

### Brief Description of Drawings

FIG. 1 is a front view of a forceps device according to an embodiment.
FIGS. 2A and 2B are schematic views for explaining a state in which a joint cover comes off a base cap.
FIG. 3 is an enlarged view of a structure around an enlarged-diameter portion of the forceps device according to the embodiment.
FIG. 4 is a schematic view for explaining a difference in deflection depending on a position at which an end of a trocar comes in contact with the joint cover.

### Description of Embodiments

The present invention will now be described on the basis of an embodiment with reference to the drawings. Components, members, and processes that are the same as or equivalent to each other illustrated in the drawings are represented by the same reference numerals, and redundant explanation will not be repeated where appropriate. The embodiment is not to limit the invention, but is an example, and any feature or any combination of features described in the embodiment is not necessarily essential to the invention.

### [Forceps device]

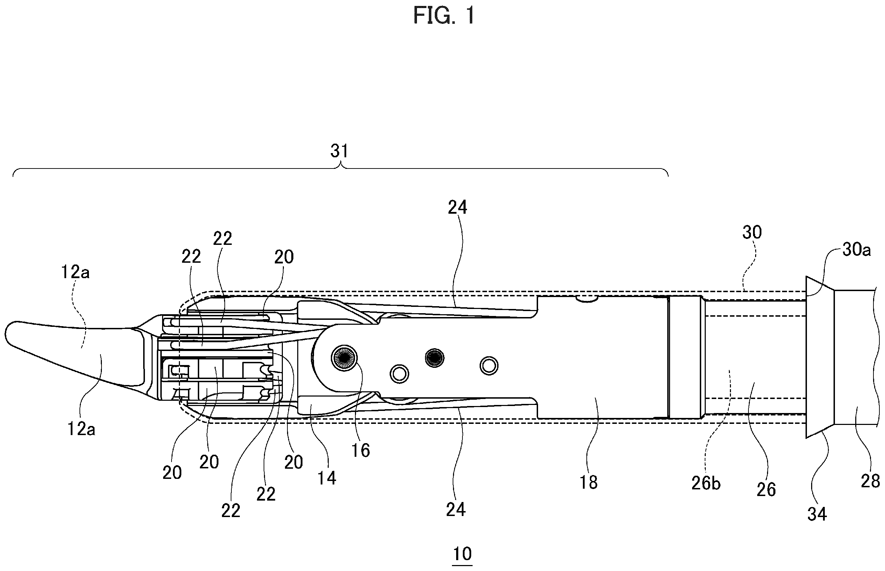

FIG. 1 is a front view of a forceps device according to an embodiment. The forceps device 10 for medical use illustrated in FIG. 1 includes a pair of grasping portions 12a and 12b, a support 14 that holds the pair of grasping portions 12a and 12b, a first rotating shaft 16 that turnably supports the support 14, a base member 18 that holds the first rotating shaft 16, four guide pulleys (not illustrated) arranged coaxially with the first rotating shaft 16, four jaw pulleys 20 supported coaxially with the pair of grasping portions 12a and 12b, four wires 22 running over the four guide pulleys and the four jaw pulleys 20, and two wires 24 for turning the support 14 about the first rotating shaft 16.

The base member 18 is connected with a front end of a base cap 26, which is a columnar member. A rear end of the base cap 26 is connected with a pipe 28. The forceps device 10 according to the embodiment is covered with a joint cover 30 having a cylindrical shape from the support 14 to the rear end of the base cap 26. Thus, foreign substances hardly enter gaps between components such as wires and pulleys during surgery. Note that the joint cover 30 may be designed to cover part of the grasping portions 12a and 12b in addition to the support 14. Alternatively, for a forceps device that transmits electricity to the grasping portions 12a and 12b for hemostasis of patient's tissue, an insulating joint cover 30 is used.

The base cap 26 is a plastic part having a cylindrical shape (columnar shape) and made of an engineering plastic material or the like. This achieves both the strength and the insulation property of the part. Specifically, examples of the material include, but not limited to, polyetheretherketone (PEEK) or polyetherimide (PEI) .

For the pipe 28, such a material as carbon fiber reinforced plastic (CFRP) or glass fiber reinforced plastic (GFRP) is used, for example. Examples of the material for the joint cover 30 include, but are not limited to, butyl rubber, silicone rubber, and/or polyethylene.

As described above, the forceps device 10 according to the embodiment includes a distal end part 31 including the grasping portions 12a and 12b, which constitute a treatment part for treating an object (a body site) to be operated, the base cap 26 connected to the distal end part 31, the pipe 28 connected to the base cap 26 on a side thereof opposite the side with which the distal end part 31 is connected, and the joint cover 30 mounted over the distal end part 31 to the base cap 26.

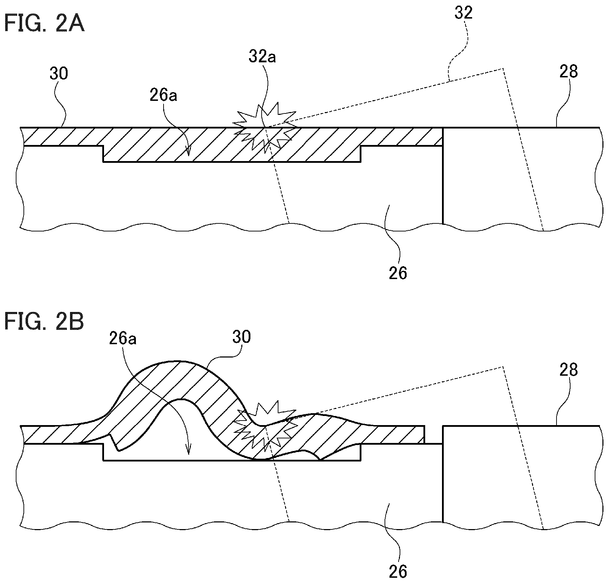

FIGS. 2A and 2B are schematic views for explaining a state in which the joint cover comes off the base cap. The forceps device 10 is inserted into a patient's body through a trocar 32 in laparoscopic surgery. The inserted forceps device 10 may incline or slide inside a patient's body. Thus, part of the joint cover 30 being caught on an end 32a of the trocar 32 (FIG. 2A) rises up at a recess 26a of the base cap 26 (FIG. 2B) as the forceps device 10 moves in a direction in which the forceps device 10 is removed from the trocar 32. As a result, an area of contact between the joint cover 30 and the recess 26a of the base cap 26 decreases, which reduces the friction therebetween, and therefore makes the joint cover 30 easier to be shifted relative to the base cap 26.

If a force is further applied to the joint cover 30 in this state from the trocar 32, the joint cover 30 may eventually come off the forceps device 10. The forceps device 10 according to the embodiment therefore has an enlarged-diameter portion (large-diameter portion) having a larger diameter than the pipe 28 and the base cap 26.

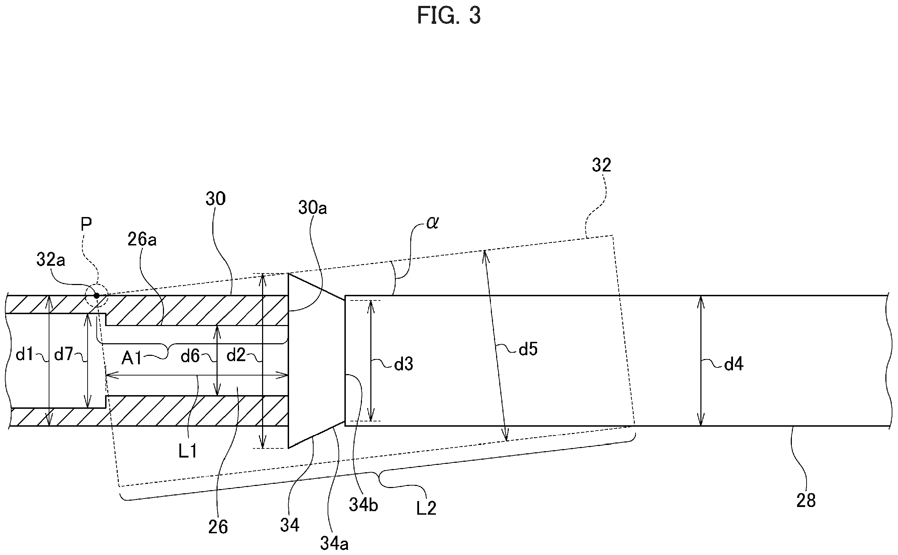

FIG. 3 is an enlarged view of a structure around the enlarged-diameter portion of the forceps device according to the embodiment. As illustrated in FIG. 3, the enlarged-diameter portion 34 has a diameter d2 that is larger than the diameter d1 of an end face 30a, which is on a side closer to the pipe, of the joint cover 30 mounted over the base cap 26. According to the embodiment, the diameter d1 is set within a range from 4.0 to 15.0 mm (or preferably within a range of 8.7±1.5 mm), and the diameter d2 is set within a range from 4.1 to 15.1 mm (or preferably within a range of 9.611.5 mm). In addition, the enlarged-diameter portion 34 is located at a position covering the end face 30a. In other words, the enlarged-diameter portion 34 located at a position covering the end face 30a can hide the end face 30a as viewed from the end 32a of the trocar 32 when the forceps device 10 inserted in the trocar 32 is being removed.

The enlarged-diameter portion 34 provided as described above makes the end 32a of the trocar 32 less likely to get caught on the end face 30a of the joint cover 30 when the forceps device 10 is being removed from the trocar 32 or inserted into the trocar 32.

The enlarged-diameter portion 34 has a tapered shape 34a with its diameter decreasing from the base cap 26 side toward the pipe 28 side. As a result, the angle α between the axis of the trocar 32 and the axis of the forceps device 10 when the forceps device 10 inserted in the trocar 32 is being removed is an acute angle. Consequently, if the end 32a of the trocar 32 comes in contact with the joint cover 30, the contact position P can be shifted from the end face 30a of the joint cover 30 toward the distal end side.

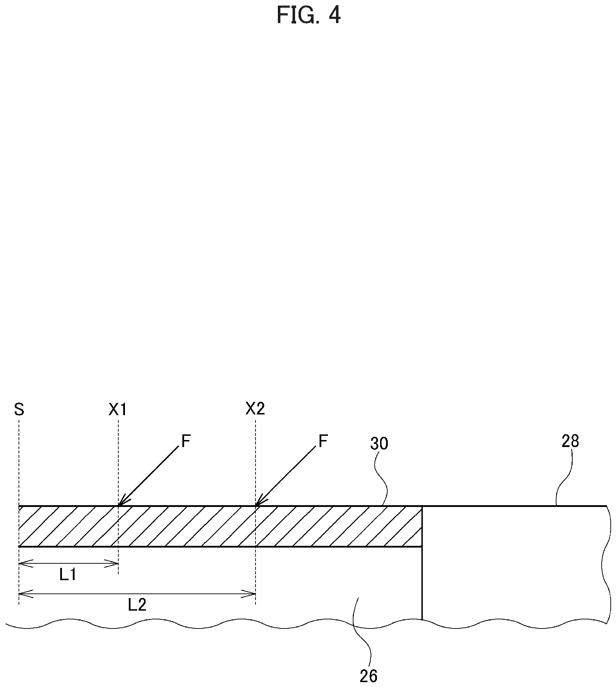

FIG. 4 is a schematic view for explaining the difference in deflection depending on the position at which the end of the trocar comes in contact with the joint cover. As illustrated in FIG. 4, a contact point X1 is at a distance L1 from a reference position S on the distal end side, and a contact point X2 is at a distance L2 (L2>L1) from the reference position S on the distal end side. In this case, the joint cover 30 is more stiff at the contact point X2. When the contact position P is not at the end face 30a of the joint cover 30 but is moved toward the distal end side as described above, the joint cover 30 is less easily bent at least at a part A1, which is on the side closer to the pipe 28 with respect to the contact position P. As a result, the joint cover 30 is less likely to come off the base cap 26 due to static frictional force at the part A1.

The diameter d3 of an end 34b, which is adjacent to the pipe, of the tapered shape 34a of the enlarged-diameter portion 34 is smaller than the diameter d4 of the pipe 28. In the embodiment, the diameter d3 is set within a range from 3.9 to 14.9 mm (or preferably within a range of 8.011.5 mm), and the diameter d4 is set within a range from 4.0 to 15.0 mm (or preferably within a range of 8.4±1.5 mm). As a result, the end 32a of the trocar 32 is less likely to get caught on the end 34b, which is adjacent to the pipe, of the enlarged-diameter portion 34 when the forceps device 10 inserted in the trocar 32 is being removed.

The enlarged-diameter portion 34 may be provided as part of the base cap 26, part of the pipe, or a separate component in the forceps device 10. This makes the end 32a of the trocar 32 less likely to get caught on the end face 30a of the joint cover 30 around the region between the base cap 26 and the pipe 28.

The diameter d2 of the enlarged-diameter portion 34 on the base cap 26 side is smaller than the inner diameter d5 of the trocar into which the forceps device 10 is inserted. This enables the forceps device 10 to be inserted into the trocar 32. According to the embodiment, the inner diameter d5 is set within a range from 5.0 to 15.0 mm (or preferably within a range of 10 to 15 mm), and the diameter d2 of the enlarged-diameter portion 34 is set within a range of 99% of the inner diameter d5 of the trocar 32 or smaller. In addition, the effective length L2 of the trocar 32 is set within a range from 30 to 200 mm (or preferably within a range of 30 to 100 mm).

If the diameter d2 is too large, the change in the posture of the forceps device 10 relative to the trocar 32 is too small. In contrast, if the diameter d2 is too small, the inclination α of the trocar 32 relative to the forceps device 10 tends to be large, and the amount (contact load) by which the end 32a of the trocar 32 bites into the joint cover 30 at the contact position P becomes larger. Furthermore, the contact position P becomes likely to be closer to the end face 30a of the joint cover 30. The position at which the enlarged-diameter portion 34 is provided and the diameter d2 are therefore preferably set so that the inclination α relative to the trocar 32 into and from which the forceps device 10 is inserted and removed is an acute angle.

Note that the enlarged-diameter portion 34 supporting an inner wall of the trocar 32 partly bears the load applied when the trocar 32 comes in contact with the joint cover 30. This makes the amount by which the end 32a of the trocar 32 bites into the joint cover 30 smaller. In addition, the presence of the enlarged-diameter portion 34 enables the position at which the end 32a of the trocar 32 comes into contact with the joint cover 30 to be shifted toward the distal end side with respect to the end face 30a of the joint cover 30. As a result, even if the trocar 32 comes into contact with the joint cover 30, the trocar 32 touches a position X1, which is closer to the distal end side and has a higher stiffness, rather than a position X2, which is closer to the end face 30a of the joint cover 30 and has a lower stiffness (see FIG. 4). This prevents or minimizes deflection of the joint cover 30.

Furthermore, when the trocar 32 comes in contact with the position X2, the friction between the base cap 26 and the joint cover 30 generated over an area from the position X2 to the end face 30a acts as a reaction force. Thus, when the trocar 32 comes in contact with the position X1, which is closer to the distal end side than the position X2, the friction between the base cap 26 and the joint cover 30 generated over the area from the contact position to the end face 30a is larger, which makes the joint cover 30 less likely to come off.

The base cap 26 according to the embodiment has a through-hole 26b through which wires 22 and wires 24 for causing the grasping portions 12a and 12b, which constitute the treatment part, to perform such operation as opening/closing and turning. The length L1 of the recess 26a formed on a portion of the base cap 26 close to the pipe 28 is set within a range of 7.0±3 mm, and the diameter d6 of a small-diameter portion of the recess 26a is set within a range of 3.0 to 11.9 mm (or preferably within a range of 7.2±1.5 mm) . In addition, the diameter d7 of a large-diameter portion of the base cap 26 is set within a range of 3.1 to 12.0 mm (or preferably within a range of 7.8±1.5 mm).

Furthermore, the diameter d2 of the enlarged-diameter portion 34 is preferably set so that the contact position P at which the end 32a of the trocar 32 comes into contact with the joint cover 30 is on the side close to the distal end side with respect to the recess 26a of the base cap 26. In this case, part of the joint cover 30 fitted in the recess 26a close to the pipe 28 with respect to the contact position P is caught by the stepped portion, which makes the joint cover 30 still less likely to come off the base cap 26.

Note that the enlarged-diameter portion 34 may be a plastic part made of a material such as an engineering plastic material. This achieves both the strength and the insulation property of the part. Specifically, examples of the material include, but are not limited to, polyetheretherketone (PEEK) or polyetherimide (PEI).

In addition, while an example of a forceps device having an annular recess 26a around the outer circumference of the base cap 26 has been described in the embodiment, a forceps device without the recess 26a, that is, with no stepped portion corresponding to a side wall of the recess 26a is also applicable.

While the present invention has been described above with reference to an embodiment, the present invention is not limited to the embodiment, and any combination or substitution of components in the embodiment as appropriate is included in the present invention. In addition, modifications such as combinations, changes in the order of processes, and various changes in design in the embodiment may be made on the embodiment on the basis of knowledge of a person skilled in the art, and such modified embodiments may be within the scope of the present invention.

### Industrial Applicability

The present invention makes a cover less likely to come off a forceps device.

### Reference Signs List

- 10: forceps device
- 12a, 12b: grasping portion
- 14: support
- 16: first rotating shaft
- 18: base member
- 20: jaw pulley
- 22: wire
- 24: wire
- 26: base cap
- 26a: recess
- 26b: through-hole
- 28: pipe
- 30: joint cover
- 30a: end face
- 31: distal end part
- 32: trocar
- 32a: end
- 34: enlarged-diameter portion
- 34a: tapered shape
- 34b: end

## Claims

1. A forceps device comprising:
a distal end part including a treatment part for treating an object;
a columnar member connected to the distal end part;
a pipe connected to the columnar member on a side thereof opposite the side with which the distal end part is connected;
a cover mounted over the distal end part to the columnar member; and
an enlarged-diameter portion having a larger diameter than an end face, on a side closer to the pipe, of the cover mounted over the columnar member,
wherein the enlarged-diameter portion is located at a position covering the end face.

2. The forceps device according to claim 1,
wherein the enlarged-diameter portion has a tapered shape with its diameter decreasing from an end adjacent to the columnar member toward an end adjacent to the pipe.

3. The forceps device according to claim 2,
wherein a diameter of the enlarged-diameter portion at the end of the tapered shape adjacent to the pipe is smaller than a diameter of the pipe.

4. The forceps device according to any one of claims 1 to 3,
wherein the enlarged-diameter portion is provided at the columnar member or the pipe.

5. The forceps device according to any one of claims 1 to 4,
wherein the diameter of the enlarged-diameter portion on the columnar member side is smaller than an inner diameter of a trocar into which the forceps device is inserted.

6. The forceps device according to any one of claims 1 to 5, further comprising:
a wire for causing the treatment part to operate,
wherein the columnar member has a through-hole through which the wire extends.
